# EUROPÄISCHE PATENTSCHRIFT

(11) **EP 4 104 025 B1**
(45) Veröffentlichungstag und Bekanntmachung des Hinweises auf die Patenterteilung: **02.07.2025**
(21) Anmeldenummer: 21705160.6
(22) Anmeldetag: 11.02.2021
(51) Int. Cl.: G05B 19/401, G05B 19/418

(54) **VERFAHREN UND VORRICHTUNG ZUR IDENTIFIKATION VON WERKSTÜCKEN**
METHOD AND DEVICE FOR IDENTIFYING WORKPIECES
PROCÉDÉ ET DISPOSITIF D'IDENTIFICATION DE PIÈCES

(30) Priorität: 12.02.2020 DE 102020103580
(43) Veröffentlichungstag der Anmeldung: 21.12.2022
(73) Patentinhaber: HOMAG GmbH, 72296 Schopfloch (DE)
(72) Erfinder: HOMEIER, Achim, 72285 Pfalzgrafenweiler (DE)
(74) Vertreter: Hoffmann Eitle
(86) Internationale Anmeldenummer: PCT/EP2021/053313
(87) Internationale Veröffentlichungsnummer: WO 2021/160729

(56) Entgegenhaltungen:
- DE-A1- 102017 207 992

## Beschreibung

### Technisches Gebiet

Die Erfindung betrifft ein Verfahren und eine Vorrichtung zur Identifikation von Werkstücken während eines Bearbeitungsprozesses, wobei die Werkstücke aus Holz, Holzwerkstoffen oder dergleichen bestehen.

### Stand der Technik

Im Allgemeinen müssen Werkstücke, welche aus Holz, Holzwerkstoffen oder dergleichen bestehen, während eines Bearbeitungsprozesses erfasst und gekennzeichnet werden. Im Bereich der Möbel- und Bauelementindustrie werden in den Verfahren zum Kennzeichnen von Werkstücken der eingangs genannter Art oftmals Kennzeichnungsträger wie Barcodes, Transponder (RFID) und Mikrochips verwendet.

Die Kennzeichnung dient dabei meist der eindeutigen Identifikation eines Werkstücks im Produktionsverlauf. Zusätzlich können auch Werkstück-Eigenschaften wie zum Beispiel Dimension, Farbe und Werkstoff auf dem Kennzeichnungsträger hinterlegt werden. Durch Verknüpfung der Informationen auf dem Kennzeichnungsträger mit entsprechenden Routinen im Bearbeitungsprozess kann die Bearbeitung der Werkstücke weitgehend automatisiert erfolgen.

Bei einer oberflächlichen Anbringung von Kennzeichnungsträgern an einem Werkstück besteht allerdings die Gefahr, dass diese im weiteren Bearbeitungsprozess verloren gehen oder beschädigt werden. Weiterhin müssen die Kennzeichnungsträger nach dem Bearbeitungsprozess oftmals entfernt werden, da sie zum Beispiel aus ästhetischen oder produktionstechnischen Gesichtspunkten störend sein können. Außerdem sind das Anbringen und Erfassen der Kennzeichnungsträger jeweils mit zusätzlichen apparativen Maßnahmen verknüpft.

So offenbart EP2230626 ein Verfahren zur Kennzeichnung von Massivplattenwerkstücken durch Anbringen eines RFID-Tags insbesondere in einer zu fertigenden Ausnehmung des Werkstücks, wobei der eingebrachte RFID-Tag mit einer Beschichtung zum Schutz gegen die Umgebung versehen wird. DE102014208746 offenbart ein weiteres Verfahren zum Kennzeichnen von Werkstücken, mit den Schritten Aufbringen einer codierbaren Beschichtung, insbesondere Folie, Kante und/oder Klebstoffes, auf eine Oberflache des Werkstücks, sowie reversible oder irreversible Codierung der Beschichtung durch eine Codiereinheit derart, dass die Codierung durch eine Leseeinheit wieder ausgelesen werden kann.

Bei der Bearbeitung von Werkstücken, welche aus Holz, Holzwerkstoffen oder dergleichen bestehen, treten weiterhin Probleme auf, wenn sich beispielsweise durch äußere Einflüsse wie Temperaturen die Abmessungen der Werkstücke verändern und anschließend die Werkstücke ungenau in einer Bearbeitungsmaschine positioniert sind und dadurch der Bearbeitungsschritt unsachgemäß ausgeführt wird. Mit Hilfe elektronischer Erfassungseinrichtungen können jedoch Werkstück-Eigenschaften vor der Bearbeitungsmaschine erfasst werden und der Bearbeitungsschritt entsprechend angepasst werden.

EP2308659 offenbart eine Vorrichtung zum Bearbeiten von Werkstücken, welche eine Fördereinrichtung zum Fördern der Werkstücke und eine elektronische Erfassungseinrichtung zum Erfassen einer Position einer optischen und/oder dreidimensionalen Struktur der auf der Fördereinrichtung geförderten Werkstücke.

Bei unsachgemäßer Ausführung eines Bearbeitungsschritts kann der nachfolgende Bearbeitungsschritt nachteilig beeinflusst werden oder das fehlerhaft bearbeitete Werkstück verlässt unbemerkt den Bearbeitungsprozess.

Ein weiteres Beispiel eines bisher bekannten Verfahrens zur Identifikation von Werkstücken kann DE 10 2017 207 992 A1 entnommen werden.

### Darstellung der Erfindung

Der Erfindung liegt die Aufgabe zugrunde, ein Verfahren und eine dafür vorgesehene Vorrichtung zur Identifikation von Werkstücken bereit zu stellen, welche eine Steuerung und Qualitätskontrolle der Werkstücke im Bearbeitungsprozess ermöglichen, wobei der dafür notwendige apparative Aufwand gering gehalten werden soll.

Erfindungsgemäß wird diese Aufgabe durch ein Verfahren zur Identifikation von Werkstücken mit den Merkmalen des Patentanspruchs 1 und durch eine Vorrichtung zur Ausführung des Verfahrens nach dem Patentanspruch 10 gelöst. In den Unteransprüchen finden sich vorteilhafte Ausgestaltungen und Verbesserungen der Erfindung.

Der Erfindung liegt dabei insbesondere die Erkenntnis zugrunde, dass bei der Identifikation und Qualitätskontrolle von Werkstücken in einem Bearbeitungsprozess synergistische Effekte nutzbar sind, was den hierfür ursprünglich notwendigen apparative Aufwand reduziert. Weiterhin wurde erkannt, dass zur Steuerung und Überwachung eines Bearbeitungsprozesses mit unterschiedlichen Werkstücken nicht notwendigerweise jedes Werkstück individuell gekennzeichnet werden muss. Zu diesem Zweck werden bei dem vorliegenden Verfahren die Werkstücke anhand ihrer Werkstück-Eigenschaften identifiziert. Entsprechen die Werkstück-Eigenschaften zur Werkstück-Identifizierung den Werkstück-Eigenschaften zur Werkstück-Prüfung, können beide Vorgänge mit demselben Erfassungssystems durchgeführt werden. Als besonders geeignet hat sich in diesem Zusammenhang die Erfassung von geometrischen und/oder physikalischen Werkstück-Eigenschaften herausgestellt.

Unter Nutzung dieser Erkenntnisse stellt die Erfindung ein Verfahren zur Identifikation von Werkstücken gemäß Anspruch 1 bereit.

Die Erfindung sorgt somit dafür, dass eine Identifizierung des Werkstücks ausschließlich anhand der Werkstück-Eigenschaften selbst erfolgen kann. Die Identifizierungsdaten des Werkstücks sind folglich untrennbar mit dem Werkstück verknüpft. Das Werkstück ist dadurch jederzeit im Bearbeitungsprozess zu identifizieren. Eine zusätzliche Anbringung von Kennzeichnungsträgern ist demzufolge nicht notwendig.

Weiterhin ergibt sich daraus ein dynamischer Bearbeitungsprozess, da ein Bearbeitungsschritt entsprechend der vorliegenden Werkstück-Eigenschaften des zu bearbeitenden Werkstücks situativ angepasst wird und nicht auf definierte Daten eines Kennzeichnungsträgers beschränkt ist.

Darüber hinaus ergibt sich der Vorteil, dass die erfassten Werkstück-Eigenschaften, welche für die Zuordnung zu einer entsprechenden Werkstück-Kategorie genutzt werden, auch gleichzeitig die Eigenschaften eines Werkstücks sind, 35 welche auf möglich Abweichungen von einer Norm geprüft werden sollen. Der synergistische Effekt besteht folglich darin, dass die erfassten Werkstück-Eigenschaften zur Werkstück-Identifizierung und die zu prüfenden Werkstück-Eigenschaften übereinstimmen. Der apparative Aufwand bei der Erfassung von Werkstück-Eigenschaften zur Werkstück-Identifizierung und Werkstück-Prüfung kann auf dasselbe Erfassungssystem beschränkt werden.

Bevorzugt wird das Verfahren gemäß der Erfindung derart ausgeführt, dass mindestens eine zu ändernde Werkstück-Eigenschaft nach dem Bearbeitungsschritt erfasst wird und auf dieser Grundlage ein nächster Prozessschritt bestimmt wird. Auf diese Weise wird sichergestellt, dass das Werkstück entsprechend dem Bearbeitungsschritt ordnungsgemäß bearbeitet wird. Dabei ist es vorteilhaft, dass der nächste Prozessschritt vom Bearbeitungsergebnis des vorherigen Bearbeitungsschritts abhängig ist.

Weiterhin ist es vorteilhaft, dass Informationen über den Bearbeitungserfolg des Werkstücks generiert und mit diesem assoziiert sind. Mit Hilfe der zusätzlich gewonnen Informationen über den Bearbeitungserfolg der Werkstücke kann der Bearbeitungsprozess effektiver gesteuert werden. Besonders bevorzugt ist das erfindungsgemäße Verfahren wonach im nächsten Prozessschritt das Werkstück weiter bearbeitet wird oder aus dem Bearbeitungsprozess ausgeschleust wird. Hierbei liegt der Vorteil, dass nur sachgemäß bearbeitete Werkstücke im Bearbeitungsprozess verbleiben und nachfolgende Bearbeitungsschritte nicht durch unsachgemäße bearbeitete Werkstücke beeinflusst werden. Werkstücke mit irreversiblen Bearbeitungsfehlern bleiben im Bearbeitungsprozess nicht unerkannt und werden ausgeschleust. Hingegen können Werkstücke mit reversiblen Bearbeitungsfehlern einer zusätzlichen Bearbeitung in einem weiteren Bearbeitungsschritt zugeführt werden.

In einer weiteren vorteilhaften Ausgestaltung des Verfahrens der Erfindung werden vor dem Bearbeitungsschritt zusätzlich die mindestens eine zu ändernde Werkstück-Eigenschaft erfasst, und das Werkstück wird entsprechend der zugewiesenen Werkstück-Kategorie und der zusätzlich erfassten zu ändernden Werkstück-Eigenschaft bearbeitet.

Durch die Erfassung mindestens einer zu ändernden Werkstück-Eigenschaft vor dem Bearbeitungsschritt ist es möglich, dass ein Werkstück einen Bearbeitungsschritt auch mehrmals durchlaufen kann. Dies hat den Vorteil, dass bei wiederholtem Durchlaufen eines Bearbeitungsschritts jeweils unterschiedliche Werkstück-Eigenschaften geändert werden können und nicht wiederholt derselbe Bearbeitungsschritt ausgeführt wird.

Das vorliegende Verfahren der Erfindung zeichnet sich insbesondere dadurch aus, dass durch Kombination einer Vielzahl an denselben und/oder unterschiedlichen Werkstück-Eigenschaften das Werkstück einer Werkstück-Kategorie zugeordnet wird.

Folglich steht abhängig von der erfassten Anzahl an denselben und/oder unterschiedlichen Werkstück-Eigenschaften eine entsprechende Anzahl an definierbaren Werkstück-Kategorien zur Verfügung, in welche die Werkstücke zugeordnet werden können. Dabei besteht der Vorteil, dass durch Erhöhung der Anzahl der erfassten Werkstück-Eigenschaften entsprechend die Anzahl der zuordbaren Werkstück-Kategorien erhöht werden kann.

In einer weiteren bevorzugten Ausführungsform des Verfahrens werden physikalische und/oder geometrische Werkstück-Eigenschaften erfasst. Dies können beispielsweise Gewichtsangaben, Abmessungen, Farben, oder dreidimensionale Strukturen der Werkstücke sein.

Die physikalischen und/oder geometrischen Werkstück-Eigenschaften bieten eine Vielzahl an unterschiedlichen zu erfassenden Wertemengen. Dies ermöglicht einen Datenpool mit einer Vielzahl an Werkstück-Eigenschaften zur Verfügung zu stellen und daraus eine Vielzahl an unterschiedlichen Werkstück-Kategorien zu kombinieren, auf deren Basis die Werkstücke identifiziert werden.

In einer weiteren bevorzugten Ausführungsform werden die zu erfassenden Werkstück-Eigenschaften und die Werkstück-Kategorien in einem Datenpool definiert.

Aus einer Vielzahl an Werkstück-Eigenschaften und Werkstück-Kategorien wird dadurch ein Datenpool an Werkstück-Eigenschaften und Werkstück-Kategorien definiert. Es besteht der Vorteil, dass zu unterschiedlichen Bearbeitungsprozessen mit unterschiedlichen Werkstücken flexibel verschiedene Datenpools an Werkstück-Eigenschaften und Werkstück-Kategorien festgelegt werden können, auf deren Basis es möglich ist, unterschiedliche Werkstücke zu identifizieren.

Besonders bevorzugt ist bei dem vorliegenden Verfahren, bei dem die während des Bearbeitungsprozess erfassten Werkstück-Eigenschaften zusätzlich im Datenpool gespeichert werden.

Auf diese Weise wird ein Datenpool an Werkstück-Eigenschaften und generiert, welcher den Werkstücken entspricht, die sich im Bearbeitungsprozess befinden. Dies hat den Vorteil, dass Echtzeit-Daten über die im Bearbeitungsprozess befindlichen Werkstücke und deren Eigenschaften verfügbar sind.

Bevorzugt wird weiterhin, dass ein Prozessleitsystem auf den Datenpool zugreift und den Bearbeitungsprozess steuert.

Dies ermöglicht, dass einzelne Bearbeitungsschritte des Bearbeitungsprozesse aufeinander abgestimmt werden können und der Bearbeitungsprozess optimal ausgelastet werden kann. Ferner bietet das Prozessleitsystem dem Benutzer einen Überblick über die gerade im Bearbeitungsprozess befindlichen Werkstücke und ermöglicht es, einen übergeordneten Produktionsprozess flexibel zu gestalten.

Die vorliegende Erfindung stellt weiterhin eine Vorrichtung zur Durchführung des Verfahrens bereit, umfassend: mindestens ein Erfassungssystem, das vor einer Werkstück-Bearbeitungseinheit eingerichtet ist, um Werkstückeigenschaften zu erfassen, die zur Zuordnung einer Werkstück-Kategorie notwendig sind und mindestens eine Werkstück-Bearbeitungseinheit, die eingerichtet ist, um das Werkstück entsprechend einer zugeordneten Werkstück-Kategorie zu bearbeiten.

Die Erfindung sorgt somit dafür, dass vor der Bearbeitungseinheit auf Basis der zugeordneten Werkstück-Kategorie festgelegt wird, welcher Bearbeitungsschritt durchgeführt wird. Dies hat den Vorteil, dass auf einen Kennzeichnungsträger verzichtet werden kann. Das Erfassungssystem ermöglicht neben der Erfassung der Werkstück-Eigenschaften zur Identifizierung der Werkstücke zusätzlich eine Prüfung dieser Werkstück-Eigenschaften. Dies hat den Vorteil, dass der sich der apparative Aufwand auf ein Erfassungssystem beschränkt.

Ein Bearbeitungsprozess mit einzelnen Bearbeitungsschritten kann außerdem dynamisch erfolgen, da ein Bearbeitungsschritt situativ entsprechend der erfassten Werkstück-Eigenschaften angepasst wird und nicht auf die Daten eines Kennzeichnungsträgers angewiesen ist.

In einer bevorzugten Ausführungsform der Vorrichtung wird mindestens ein Erfassungssystem nach der Werkstück-Bearbeitungseinheit eingerichtet, um die zu ändernde Werkstück-Eigenschaft zu erfassen.

Auf diese Weise wird sichergestellt, dass das Werkstück entsprechend dem Bearbeitungsschritt ordnungsgemäß bearbeitet wird und zusätzliche Informationen über den Bearbeitungserfolg des Werkstücks generiert werden.

In einer vorteilhaften Ausgestaltung der Vorrichtung ist mindestens ein Erfassungssystem vor der Bearbeitungseinheit eingerichtet, um die zu ändernde Werkstück-Eigenschaft zu erfassen.

Auf diese Weise wird erfasst, ob in der Bearbeitungseinheit gemäß der zugewiesen Werkstück-Kategorie eine Bearbeitung durchgeführt werden muss. Dies hat den Vorteil, dass eine Bearbeitungseinheit unterschiedliche Bearbeitungsschritte an einem Werkstück ausführen kann.

Weiterhin weist in einer bevorzugten Ausführungsform der Vorrichtung das Erfassungssystem ein und/oder mehrere aktive und/oder passive Sensoren auf, insbesondere mechanische, thermoelektrische, resistive, piezoelektrische, kapazitive, induktive, optische, akustische oder magnetische Sensoren. Damit kann eine Vielzahl an geometrischen und/oder physikalischen Werkstück-Eigenschaften erfasst werden. Es ist dabei vorteilhaft, dass die Sensoren entsprechend der Eigenschaften der zu bearbeitenden Werkstücke individuell ausgewählt werden können.

In einer besonders bevorzugten Ausführungsform der Vorrichtung ist ein Weichenelement nach der Bearbeitungseinheit eingerichtet, um das Werkstück auszuschleusen oder einem weiteren Bearbeitungsschritt zuzuführen.

Durch das Weichenelement können unsachgemäß bearbeitete Werkstücke entweder aus dem Bearbeitungsprozess ausgeschleust oder einem Bearbeitungsschritt erneut zugeführt werden. Dies hat den Vorteil, dass diese Werkstücke nicht sofort per Hand entfernt oder an anderer Stelle im Bearbeitungsprozess umgesetzt werden müssen. Dadurch wird verhindert, dass unsachgemäß bearbeitete Werkstücke die Kapazitäten von weiteren Bearbeitungsschritten in Anspruch nehmen.

Weiter bevorzugt weist die Vorrichtung ein Prozessleitsystem auf, um den Bearbeitungsprozess anhand des gespeicherten Datenpools der erfassten Werkstück-Eigenschaften und der zugeordneten Werkstück-Kategorien zu steuern.

Dies ermöglicht, dass einzelne Bearbeitungsschritte des Bearbeitungsprozesses aufeinander abgestimmt werden können und der Bearbeitungsprozess dadurch optimal ausgelastet werden kann. Ferner bietet das Prozessleitsystem dem Benutzer einen Überblick über die gerade im Bearbeitungsprozess befindlichen Werkstücke und ermöglicht dadurch einen übergeordneten Produktionsprozess flexibel zu gestalten.

### Kurze Beschreibung der Zeichnungen

Weitere Merkmale und Vorteile einer Vorrichtung, einer Verwendung und/oder eines Verfahrens ergeben sich aus der nachfolgenden Beschreibung von Ausführungsformen unter Bezugnahme auf die beiliegenden Zeichnungen. Von diesen Zeichnungen zeigt:
Figur 1: eine schematische Zeichnung einer Vorrichtung zur Identifikation von Werkstücken in einem Bearbeitungsprozess;
Figur 2: eine schematische Zeichnung einer Vorrichtung zur Identifikation einer Türe und einer Tischplatte in einem Bearbeitungsprozess, welcher beispielhaft die Bearbeitungsschritte Lackieren und Kantenanleimen beinhaltet.

### Beschreibung von Ausführungsformen

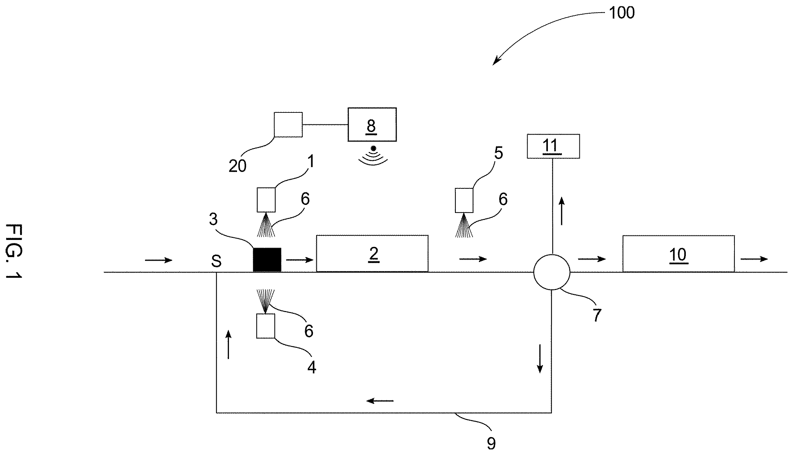

Figur 1 zeigt eine schematische Zeichnung einer Vorrichtung 100 zur Identifikation von Werkstücken 3 in einem Bearbeitungsprozess gemäß einer ersten Ausführungsform der Erfindung.

Die Werkstücke bestehen aus Holz, Holzwerkstoffen, Kunststoffen, Metall oder dergleichen. Die Werkstücke 3 sind insbesondere bekannt aus der Möbelfertigung, der Objekteinrichtung, im Fahrzeug- und Schiffsbau, im Hausbau und Innenausbau von Häusern, und dergleichen.

Vorrichtung 100 weist eine oder mehrere Bearbeitungseinheiten zum Bearbeiten der Werkstücke auf. Als Bearbeitungseinheiten sind beispielsweise die aus der Praxis bekannten Bearbeitungs- und Beschichtungsmaschinen, wie Format- und Kantenanleimmaschinen, Lackiermaschinen, Breitbandschleifmaschinen, Hobelmaschinen, Bohr- und Dübeleintreibmaschinen, Plattenaufteilsägen, und dergleichen vorgesehen.

Insbesondere ist die gezeigte Vorrichtung dergestalt, dass zwei Erfassungssysteme 1, 4 vor und ein Erfassungssystem 5 nach der Bearbeitungseinheit 2 eingerichtet sind.

Die Erfassungssysteme 1, 4, 5 sind mit unterschiedlichen Sensoren wie beispielsweise mechanischen, thermoelektrischen, resistiven, piezoelektrischen, kapazitiven, induktiven, optischen, akustischen oder magnetischen Sensoren ausgestattet.

In der vorliegenden Ausführungsform ist eine Fördereinrichtung 9 so eingerichtet, dass diese einen geschlossenen Förderkreislauf eines Werkstücks 3 ermöglicht.

Die Fördereinrichtung 9 zum Fördern der Werkstücke 3 ist beispielsweise in Form von Förderbändern, Förderriemen, Förderketten, fahrerlosen Transportsystemen (FTS), autonomen Handlingssystemen, Robotern und dergleichen eingerichtet.

Die Werkstücke 3 werden an einer Stelle S dem Förderkreislauf neu zugeführt und mit Hilfe eines Weichenelements 7 kann geregelt werden, dass diese entweder im Förderkreislauf verbleiben, in eine externe Lagereinheit 11 ausgeschleust oder zur einer weiteren Bearbeitungsmaschine 10 transportiert werden. Das Weichenelement 7 ist vorzugsweise direkt nach dem dritten Erfassungssystem 5 angeordnet.

Darüber hinaus ist die Vorrichtung 100 so ausgeführt, dass die Stelle S, an der die Werkstücke 3 über das Weichenelement 7 zurück zur Bearbeitungseinheit 2 transportiert werden und an der unbearbeitete Werkstücke 3 in den Bearbeitungsprozess eingeschleust werden, vor dem ersten 1 und zweiten Erfassungssystem 1, 4 liegt.

Die Vorrichtung 100 ist insbesondere so eingerichtet, dass ein zentrales Prozessleitsystem 8, beispielsweise über eine drahtlose Verbindung, die einzelnen Einheiten (1, 2, 4, 5, 6, 7, 9, 11) der Vorrichtung 100 steuert. Die in Figur 1 beschriebene Vorrichtung 100 kann als eine eigene Prozess-Einheit angesehen werden. Das Prozessleitsystem 8 kann zur Steuerung des Bearbeitungsprozesses auf einen Datenpool 20 zugreifen.

In weiteren nicht gezeigten Ausführungsformen ist vorgesehen, dass ein Gesamtprozess aus einer in Reihe und/oder parallel geschalteten Prozess-Einheiten besteht, wodurch ein durchgängiger und lückenloser Bearbeitungsprozess eines Werkstücks 3 ermöglicht wird.

Nachfolgend soll nun beispielhaft das Verfahren anhand der in Figur 1 gezeigten Vorrichtung 100 beschrieben werden.

In einem ersten Schritt des beanspruchten Verfahrens werden zunächst die zu Identifikation festgelegten Werkstück-Eigenschaften des Werkstücks 3, welches an der Stelle S in den Bearbeitungsprozess eingeschleust und über die Fördereinrichtung 9 zum ersten Erfassungssystem 1 transportiert wird, mit Hilfe eines Sensors 6 des Erfassungssystems 1 erfasst.

Die zur Identifikation geeigneten Werkstück-Eigenschaften sind insbesondere geometrische und/oder physikalische Eigenschaften. Abhängig von den geometrischen und physikalischen Eigenschaften des zu bearbeitenden Werkstücks 3, ergibt sich eine Vielzahl an unterschiedlichen Werkstück-Eigenschaften, welche im vorliegenden Bearbeitungsprozess erfasst werden können. Die zu erfassenden Werkstück-Eigenschaften sind in einem Datenpool 20 definiert.

Im zweiten Verfahrensschritt wird das Werkstück 3 anhand der erfassten Werkstück-Eigenschaften eindeutig einer Werkstück-Kategorie zugeordnet und dadurch identifiziert. Eine Werkstück-Kategorien ist im Datenpool 20 durch eine Werkstück-Eigenschaft oder durch mehrere Werkstück-Eigenschaft nach dem Prinzip der Kombinatorik definiert. Erfindungsgemäß ist eine Werkstück-Kategorie durch ein Oberflächenmuster in Kombination mit einer Gewichtsangabe definiert. Insbesondere können dabei anstelle eines Einzelwertes für die Gewichtsangabe auch eine Wertemenge für die Gewichtsangabe definiert sein.

Die Zuordnung des Werkstücks 3 zu einer Werkstück-Kategorie wird insbesondere durch das Prozessleitsystem 8 ausgeführt, indem es auf den Datenpool 20 zugreift. Dabei gleicht das Prozessleitsystem 8 die mit Hilfe des Erfassungssystems 1 erfassten Werte der Werkstück-Eigenschaften des Werkstücks 3 mit den definierten Werten der Werkstück-Eigenschaften der definierten Werkstück-Kategorien ab. Stimmen die erfassten Werte der Werkstück-Eigenschaften des Werkstücks 3 mit den definierten Werten der Werkstück-Eigenschaften einer Werkstück-Kategorie überein, ordnet das Prozessleitsystem 8 das Werkstück 3 dieser Werkstück-Kategorie zu, wodurch es identifiziert ist.

Weiterhin prüft das Prozessleitsystem 8 die erfassten Werte der Werkstück-Eigenschaften des Werkstücks 3 beispielsweise auch auf mögliche Abweichungen von einer definierten Norm. In der Figur 1 ist nicht gezeigt, dass vor der Bearbeitungseinheit 2 ein weiteres Weichenelement eingerichtet sein kann, dass das Werkstück 3, falls es nicht einer Norm entspricht, aus dem Bearbeitungsprozess ausschleust.

Nach der eindeutigen Zuordnung des Werkstücks 3 zu einer Werkstück-Kategorie und positiver Prüfung der Werkstück-Eigenschaften auf Einhaltung einer Norm, wird der dritte Verfahrensschritt eingeleitet. In diesem wird das Werkstück 3 gemäß der zugeordneten Werkstück-Kategorie in der Bearbeitungseinheit 2 bearbeitet. Im Prozessleitsystem 8 sind insbesondere für jede Werkstück-Kategorie ein oder mehrere Arbeitsvorgänge durch die Bearbeitungseinheit 2 hinterlegt. Das Prozessleitsystem 8 bestimmt situativ gemäß der Werkstück-Kategorie die durchzuführende Bearbeitung des Werkstücks 3 in der Bearbeitungseinheit.

Nach der Bearbeitung des Werkstücks 3 in der Bearbeitungseinheit 2 erfasst ein drittes Erfassungssystem 5 mit Hilfe eines Sensors 6 die geänderten Werkstück-Eigenschaften des Werkstücks 3. Diese werden an das Prozessleitsystem 8 übermittelt und im Datenpool 20 gespeichert. Abhängig vom Bearbeitungsergebnis der Bearbeitungseinheit 2 steuert das Prozessleitsystem 8 die Förderung des Werkstücks 3 mit der Fördereinheit 9 über das Weichenelement 7 entweder erneut zu der ersten Bearbeitungseinheit 2, oder in eine Lagereinheit 11 oder zu einer weiteren Bearbeitungseinheit 10.

Eine wiederholte Zuführung des Werkstücks 3 zu der ersten Bearbeitungseinheit 2 erfolgt beispielsweise, wenn das Werkstück 3 nicht fachgemäß bearbeitet wurde, der Fehler reversibel ist und die Bearbeitung erneut ausgeführt werden kann. Es kann auch ein weiterer Bearbeitungsschritt mit derselben Bearbeitungseinheit 2 an einer anderen und/oder derselben Stelle am Werkstück 3 ausgeführt werden, sodass eine weitere Werkstück-Eigenschaft geändert wird. Dabei ist insbesondere die Bearbeitungseinheit 2 so ausgelegt, dass das Werkstück 3 in der Bearbeitungsmaschine 2 neu ausgerichtet werden kann.

Ist das Bearbeitungsergebnis unsachgemäß und der Fehler irreversibel oder die Bearbeitung abgeschlossen, wird das Werkstück 3 in der Lagereinheit 11 der Vorrichtung gelagert.

Wird das Werkstück 3 wiederholt der Bearbeitungseinheit 2 zugeführt, kann insbesondere mindestens ein zweites Erfassungssystem vor der Bearbeitungseinheit 2 vorgesehen sein, um die geänderten Werkstück-Eigenschaften des Werkstücks 3 zu erfassen. Der Arbeitsschritt in der Bearbeitungseinheit 2 wird vom Prozessleitsystem anhand der zugeordneten Werkstück-Kategorie und/oder geänderten Werkstück-Eigenschaften des Werkstücks 3 bestimmt und ausgeführt.

Im Datenpool 20 werden alle Daten zu bezüglich der erfassten Werkstück-Eigenschaften der Werkstücke 3 während des Bearbeitungsprozesses gespeichert. Durch den Zugriff des Prozessleitsystems 8 auf den Datenpool 20 kann der Bediener über eine Visulisierung feststellen, an welcher Stelle im Bearbeitungsprozess sich ein Werkstück 3 einer bestimmten Kategorie befindet und welcher Bearbeitungszustand vorliegt. Befinden sich unterschiedliche Werkstücke 3 im Bearbeitungsprozess, kann das Prozessleitsystem 8 auf Basis der Daten des Datenpools 8 die einzelnen Einheiten 1, 2, 4, 5, 6, 7, 9, 10, 11 regeln und den Gesamtprozess dynamisch steuern.

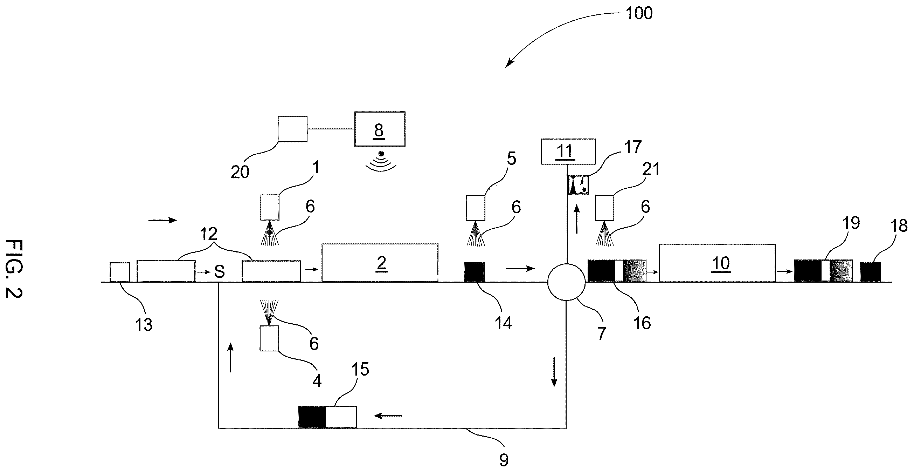

Figur 2 zeigt eine schematische Zeichnung einer Vorrichtung 100 zur Identifikation einer Türe 12 und einer Tischplatte 13 in einem Bearbeitungsprozess gemäß einer zweiten Ausführungsform der Erfindung, welcher beispielhaft die Bearbeitungsschritte Lackieren und Kantenanleimen beinhaltet.

Mit Hilfe der Figur 2 soll im Folgenden das beanspruchte Verfahren anhand eines möglichen Bearbeitungsprozesses aus der Praxis beschrieben werden.

An der Stelle S der Vorrichtung 100 werden zwei zu bearbeitende rechteckige Werkstücke, welche im hier ausgeführten Beispiel eine halbfertige Türe 12 eines Schranks und eine halbfertige Tischplatte 13 sind, in den Bearbeitungsprozess eingeschleust. Mit Hilfe einer Kamera eines ersten optischen Erfassungssystems 1 wird die Länge und die Höhe der Türe 12 (X_{Türe}, Y_{Türe}) und der Tischplatte 13 (X_{Tisch}, Y_{Tisch}) erfasst. Im Datenpool 20 ist hinterlegt, dass für eine Kombination eines bestimmten Wertebereichs [x₁,...,xₙ] bezüglich der Länge mit einem bestimmten Wertebereich [y₁,...,yₙ] bezüglich der Höhe eines Werkstücks entweder die Werkstück-Kategorie "Türe" oder "Tisch" definiert ist. Das Prozessleitsystem 8 greift auf den Datenpool 20 zu und weist das Werkstück "Türe", für welche eine Länge X_{Türe} und Höhe Y_{Türe} mit dem optischen Erfassungssystem 1 erfasst wurde, die Kategorie "Türe" zu, wenn die erfassten Werte jeweils einem Wert im entsprechenden Wertebereich, welcher für die Länge und die Höhe der Kategorie "Türe" definiert ist, entspricht. Für die Tischplatte 13 erfolgt der beschriebene Vorgang analog. Die Kategorie "Türe" und "Tisch" sind dann eindeutig mit Hilfe der zwei Wertebereiche für Länge und Höhe unterscheidbar, wenn sich mindestens eine der beiden Wertbereiche für Länge und Höhe nicht überschneidet. Die Wertbereiche werden in dem hier beschriebenen Verfahren so eng gewählt, dass diese gleichzeitig auch die Funktion erfüllen, die Tischplatte (12) und die Türe auf Abweichungen von einer Norm zu prüfen. Der Wertebereich, welcher für die Zuordnung eines Werkstücks zu einer Kategorie definiert ist, entspricht dann dem zulässigen Wertebereich für ein Normmaß.

Nachdem die unbearbeitete Tischplatte und die Türe eindeutig der Kategorie "Türe" und "Tisch" zugeordnet wurden, wird gemäß der im Prozessleitsystem 8 hinterlegte Bearbeitungsschritt für die jeweilige Werkstück-Kategorie in der Lackiermaschine 2 ausgeführt.

Das zweite optische Erfassungssystem 5 erkennt mit Hilfe einer Kamera 6 den Lackierbereich und die Musterung der Lackierung der Türe und der Tischplatte und speichert die Informationen im Datenpool 20. Auf Basis dieser Informationen veranlasst das Prozessleitsystem 8 durch Steuerung der Weiche 7, dass die teillackierte Türe 15 zurück in die Lackiermaschine 2 transportiert wird, eine Tischplatte 17 aufgrund fehlerhafter Lackierung in ein Lager 11 ausgeschleust wird und eine sachgemäß lackierte Türe 16 zur Kantenanleimmaschine 10 transportiert wird.

Das zweite optische Erfassungssystem 4 vor der Lackiermaschine 2 erfasst den teillackierten Teil der Türe 15, wobei das Prozessleitsystem 8 mit Hilfe des ersten optischen Erfassungssystem 1, welches die Höhe und Länge der Türe erfasst, die Türe (15) der Kategorie "Türe" zuordnet. Das Prozessleitsystem 8 veranlasst für die Kategorie "Türe" aufgrund der Zusatz-Information über den Lackierzustand der Türe 15, einen weiteren Lackiervorgang an der Türe durch die Lackiermaschine 2.

Das Verfahren zur Identifikation der Türe 16 und der Tischplatte 14 vor der Kantenanleimmaschine 10 erfolgt analog zum oben beschriebenen Verfahren. Durch die Kantenanleimmaschine 10 wird entsprechend der Werkstück-Kategorie eine Türe 19 und der Tischplatte 18 mit einer Kante versehen.

### Bezugszeichenliste

1 Erfassungssystem, optisches Erfassungssystem
2 Bearbeitungseinrichtung, Lackiermaschine
3 Werkstücke,
4 Erfassungssystem, optisches Erfassungssystem
5 Erfassungssystem, optisches Erfassungssystem
6 Sensor, Kamera
7 Weichenelement
8 Prozessleitsystem
9 Fördereinrichtung
10 Bearbeitungseinrichtung, Kantenanleimmaschine
11 Lagereinheit
12 Türe
13 Tischplatte
14 lackierte Tischplatte
15 teillackierte Türe
16 lackierte Türe
17 fehlerhaft lackierte Tischplatte
18 lackierte Tischplatte mit Kante
19 lackierte Türe mit Kante
20 Datenpool
21 optisches Erfassungssystem

## Patentansprüche

1. Verfahren zur Identifikation von Werkstücken (3), die zumindest teilweise aus Holz, Holzwerkstoffen oder dergleichen bestehen, in einem Bearbeitungsprozess (100) mit Bearbeitungs- und/oder Beschichtungsmaschinen, mit den Schritten:
Erfassung von Werkstück-Eigenschaften vor einem Bearbeitungsschritt mittels eines Erfassungssystems mit unterschiedlichen Sensoren,
Eindeutige Zuordnung des Werkstücks (3) zu einer Werkstück-Kategorie anhand der erfassten Werkstück-Eigenschaften, und
Bearbeitung des Werkstücks (3) in dem Bearbeitungsschritt, welcher durch die zugeordnete Werkstück-Kategorie des Werkstücks (3) bestimmt ist,
**dadurch gekennzeichnet, dass**
die Werkstück-Kategorie durch die erfassten Werkstück-Eigenschaften, nämlich ein Oberflächenmuster in Kombination mit einer Gewichtsangabe, definiert ist.

2. Verfahren nach Anspruch 1, bei dem mindestens eine zu ändernde Werkstück-Eigenschaft nach dem Bearbeitungsschritt erfasst wird und auf dieser Grundlage ein nächster Prozessschritt bestimmt wird.

3. Verfahren nach dem Anspruch 2, bei dem in dem nächsten Prozessschritt das Werkstück (3) weiter bearbeitet oder aus dem Bearbeitungsprozess (100) ausgeschleust wird.

4. Verfahren nach Anspruch 1, bei dem vor dem Bearbeitungsschritt zusätzlich die mindestens eine zu ändernde Werkstück-Eigenschaft erfasst wird und das Werkstück (3) entsprechend der zugewiesenen Werkstück-Kategorie und der zusätzlich erfassten zu ändernden Werkstück-Eigenschaft bearbeitet wird.

5. Verfahren nach einem der vorherigen Ansprüche, bei dem durch Kombination einer Vielzahl an denselben und/oder unterschiedlichen Werkstück-Eigenschaften das Werkstück (3) einer Werkstück-Kategorie zugeordnet wird.

6. Verfahren nach einem der vorherigen Ansprüche, bei dem physikalische und/oder geometrische Werkstück-Eigenschaften erfasst werden, insbesondere Gewichtsangaben, Abmessungen, Farben, oder dreidimensionale Strukturen.

7. Verfahren nach dem Anspruch 1, bei dem die zu erfassenden Werkstück-Eigenschaften und die Werkstück-Kategorien in einem Datenpool (20) definiert werden.

8. Verfahren nach dem Anspruch 7, bei dem die während des Bearbeitungsprozess erfassten Werkstück-Eigenschaften zusätzlich im Datenpool (20) gespeichert werden.

9. Verfahren nach dem Anspruch 8, bei dem ein Prozessleitsystem (8) auf den Datenpool (20) zugreift und den Bearbeitungsprozess (100) steuert.

10. Vorrichtung, eingerichtet zur Durchführung des Verfahrens nach den Ansprüchen 1 bis 9, umfassend:
Mindestens ein Erfassungssystem (1), das vor einer Werkstück-Bearbeitungseinheit (2) eingerichtet ist, um Werkstückeigenschaften zu erfassen, die zur Zuordnung einer Werkstück-Kategorie notwendig sind und
Mindestens eine Werkstück-Bearbeitungseinheit (2), die eingerichtet ist, um das Werkstück (3) entsprechend einer zugeordneten Werkstück-Kategorie zu bearbeiten.

11. Vorrichtung nach Anspruch 10, bei der mindestens ein Erfassungssystem (4) vor der Werkstück-Bearbeitungseinheit (2) eingerichtet ist, um die zu ändernden Werkstück-Eigenschaften zu erfassen.

12. Vorrichtung nach Anspruch 10 oder 11, bei dem ferner mindestens ein Erfassungssystem (5) nach der Bearbeitungseinheit (2) eingerichtet ist, um die geänderten Werkstück-Eigenschaften zu erfassen.

13. Vorrichtung nach einem der vorherigen Ansprüche, bei dem das Erfassungssystem (1, 4, 5) ein und/oder mehrere aktive und/oder passive Sensoren (6) aufweist, insbesondere mechanische, thermoelektrische, resistive, piezoelektrische, kapazitive, induktive, optische, akustische oder magnetische Sensoren.

14. Vorrichtung nach einem der Ansprüche 11 bis 13, bei dem ein Weichen-Element (7) nach dem Erfassungssystem (5) und der Bearbeitungseinheit (2) eingerichtet ist, um das Werkstück (3) auszuschleusen oder einem weiteren Bearbeitungsschritt zuzuführen.

15. Vorrichtung nach einem der vorherigen Ansprüche, bei dem das Prozessleitsystem (8) eingerichtet ist, um den Bearbeitungsprozess anhand des Datenpools (20) zu steuern.

## Claims

1. Method for identifying workpieces (3), consisting at least in part of wood, wood-based materials or the like, in a machining process (100) with machine tools and/or coating machines, said method comprising the steps of:
detecting workpiece properties before a machining step by means of a detection system having different sensors,
uniquely assigning the workpiece (3) to a workpiece category based on the detected workpiece properties, and
machining the workpiece (3) in the machining step, which is determined by the assigned workpiece category of the workpiece (3),
**characterised in that**
the workpiece category is defined by the detected workpiece properties, namely a surface pattern in combination with an indication of weight.

2. Method according to claim 1, wherein at least one workpiece property to be changed is detected after the machining step and a subsequent process step is determined on this basis.

3. Method according to claim 2, wherein the workpiece (3) is machined further in the subsequent process step or is ejected from the machining process (100).

4. Method according to claim 1, wherein the at least one workpiece property to be changed is additionally detected before the machining step and the workpiece (3) is machined according to the assigned workpiece category and the additionally detected workpiece property to be changed.

5. Method according to any of the preceding claims, wherein the workpiece (3) is assigned to a workpiece category by combining a plurality of the same and/or different workpiece properties.

6. Method according to any of the preceding claims, wherein physical and/or geometric workpiece properties are detected, in particular indications of weight, dimensions, colours or three-dimensional structures.

7. Method according to claim 1, wherein the workpiece properties to be detectd and the workpiece categories are defined in a data pool (20).

8. Method according to claim 7, wherein the workpiece properties detected during the machining process are additionally stored in the data pool (20).

9. Method according to claim 8, wherein a process control system (8) accesses the data pool (20) and controls the machining process (100).

10. Device, configured to carry out the method according to claims 1 to 9, comprising:
at least one detection system (1) which is configured upstream of a workpiece machining unit (2) to detect workpiece properties that are required for assignment to a workpiece category, and
at least one workpiece machining unit (2) which is configured to machine the workpiece (3) according to an assigned workpiece category.

11. Device according to claim 10, wherein at least one detection system (4) upstream of the workpiece machining unit (2) is configured to detect the workpiece properties to be changed.

12. Device according to claim 10 or 11, wherein, furthermore, at least one detection system (5) downstream of the machining unit (2) is configured to detect the changed workpiece properties.

13. Device according to any of the preceding claims, wherein the detection system (1, 4, 5) has one and/or more active and/or passive sensors (6), in particular mechanical, thermoelectric, resistive, piezoelectric, capacitive, inductive, optical, acoustic or magnetic sensors.

14. Device according to any of claims 11 to 13, wherein a switch element (7) downstream of the detection system (5) and machining unit (2) is configured to eject the workpiece (3) or to supply same to a further machining step.

15. Device according to any of the preceding claims, wherein the process control system (8) is configured to control the machining process based on the data pool (20).

## Revendications

1. Procédé d'identification de pièces (3) constituées au moins en partie de bois, de matériaux dérivés du bois ou similaires, dans un processus de traitement (100) avec des machines d'usinage et/ou
de revêtement, comprenant les étapes suivantes : détection des propriétés d'une pièce avant une étape de traitement au moyen d'un système de détection comprenant différents capteurs, attribution univoque de la pièce (3) à une catégorie de pièces sur la base des propriétés de pièces détectées, et
traitement de la pièce (3) lors de l'étape de traitement déterminée par la catégorie attribuée à la pièce (3), **caractérisé en ce que** la catégorie de pièce est définie par les propriétés de la pièce détectées, c'est à dire un motif de surface en combinaison avec une indication de poids.

2. Procédé selon la revendication 1, dans lequel une propriété au moins de la pièce à modifier est détectée après l'étape de traitement et une étape suivante du processus est déterminée sur cette base.

3. Procédé selon la revendication 2, dans lequel, lors de l'étape suivante du processus, la pièce (3) est traitée plus avant ou évacuée du processus de traitement (100).

4. Procédé selon la revendication 1, dans lequel, avant l'étape de traitement, au moins une propriété en plus de la pièce à modifier est détectée et la pièce (3) est traitée en fonction de la catégorie de pièce attribuée et de la propriété détectée en plus de la pièce à modifier.

5. Procédé selon l'une des revendications précédentes, dans lequel la pièce (3) est affectée à une catégorie de pièces par combinaison d'une pluralité de propriétés identiques et/ou différentes de la pièce.

6. Procédé selon l'une des revendications précédentes, dans lequel des propriétés physiques et/ou géométriques de la pièce sont détectées, en particulier des indications de poids, des dimensions, des couleurs ou des structures tridimensionnelles.

7. Procédé selon la revendication 1, dans lequel les propriétés à détecter des pièces et les catégories de pièces sont définies dans un pool de données (20).

8. Procédé selon la revendication 7, dans lequel les propriétés de la pièce détectées pendant le processus de traitement sont enregistrées en sus dans le pool de données (20).

9. Procédé selon la revendication 8, dans lequel un système de contrôle de processus (8) accède au pool de données (20) et commande le processus de traitement (100).

10. Dispositif conçu pour mettre en œuvre le procédé selon les revendications 1 à 9, comprenant :
au moins un système de détection (1) qui est installé devant une unité (2) de traitement de pièces afin de détecter les propriétés des pièces qui sont nécessaires à l'affectation à une catégorie de pièces, et
au moins une unité (2) de traitement de pièces, conçue pour traiter la pièce (3) conformément à une catégorie de pièce attribuée.

11. Dispositif selon la revendication 10, dans lequel au moins un système de détection (4) est disposé devant l'unité (2) de traitement de pièces afin détecter les propriétés à modifier des pièces.

12. Dispositif selon la revendication 10 ou la revendication 11, dans lequel au moins un système de détection (5) est en outre disposé en aval de l'unité de traitement (2) afin de détecter les propriétés modifiées des pièces.

13. Dispositif selon l'une des revendications précédentes, dans lequel le système de détection (1, 4, 5) comprend un ou plusieurs capteurs actifs et/ou passifs (6), en particulier des capteurs mécaniques, thermoélectriques, résistifs, piézoélectriques, capacitifs, inductifs, optiques, acoustiques ou magnétiques.

14. Dispositif selon l'une des revendications 11 à 13, dans lequel un élément d'aiguillage (7) est disposé après le système de détection (5) et l'unité de traitement (2) afin d'évacuer la pièce (3) ou de l'acheminer vers une étape de traitement suivante.

15. Dispositif selon l'une des revendications précédentes, dans lequel le système de contrôle de processus (8) est configuré pour contrôler le processus de traitement à l'aide du pool de données (20).
